Europäisches Patentamt

(19) European Patent Office

Office européen des brevets

(11) Publication number: **0 183 372**
**A1**

(12) **EUROPEAN PATENT APPLICATION**

(21) Application number: **85307528.1**

(22) Date of filing: **18.10.85**

(51) Int. Cl.⁴: **A 61 M 29/00**
**A 61 F 2/04**

(30) Priority: **19.10.84 US 663013**

(43) Date of publication of application:
**04.06.86 Bulletin 86/23**

(84) Designated Contracting States:
**AT BE CH DE FR GB IT LI NL SE**

(71) Applicant: **RAYCHEM CORPORATION**
**300 Constitution Drive**
**Menlo Park California 94025(US)**

(72) Inventor: **Caponigro, Dennis A.**
**48 Bunker Lane**
**Pleasanton California 94566(US)**

(74) Representative: **Benson, John Everett et al,**
**Raychem Limited Intellectual Property Law Department**
**Swan House 37-39, High Holborn**
**London WC1(GB)**

(54) **Prosthetic stent.**

㊼ A prosthetic stent (20) for internal support of a bodily duct such as a blood vessel (10) or other passage which may be collapsed for insertion into a duct and then expanded to provide active internal support for the duct. The stent (20) comprises a flexible generally tubular body of braided filaments having a first configuration (26) that is radially expanded and axially shortened and having a second configuration (28) which is radially contracted and axially lengthened and having means for biasing the tubular body from said second configuration (28) towards said first configuration (26). In one embodiment, the means for biasing the tubular body comprises the braided filaments themselves, the filaments having been annealed to a rest geometry in the first configuration (26). In another embodiment, means for biasing comprises a plurality of warp filaments that are longitudinally woven into the braided filaments to provide a frictional interface with respect to the various filaments. In yet other embodiments, elastic or heat-recoverable, shrinkable warp filaments are utilized.

FIG_3

DESCRIPTION

PROSTHETIC STENT

This invention relates to the field of interventional radiology, and more particularly to a prosthetic stent for introduction into a bodily duct, e.g., a blood vessel after an angioplasty or other procedure requiring internal support of blood vessels or similar ducts or cavities.

A significant cause of disability and death throughout the history of mankind has been cardiac disease which affects a portion of the arterial system which carries blood to and from the heart. A complication associated with cardiac disease is the acute/chronic occlusion (blocking) of blood vessels in the body and in particular the heart. Remedial measures include blood vessel resection (replacement), by-pass grafting, and angioplasty. Angioplasty appears to offer a very promising therapeutic technique in symptomatic patients having coronary and peripheral artery disease. Angioplasty is a non-surgical, minimally-invasive technique whereby a catheter with a balloon attached to one end is introduced into the body via the femoral (leg) or brachial (arm) artery and is directed to the occluded vessel. Once at the occluded site and when the catheter/balloon are in place in the stenosis (occlusion mass/plaque), the balloon is inflated using a radiopaque liquid. The inflated balloon causes the plaque to separate and, after two or three cycles of inflation and deflation, the plaque is displaced sufficiently to reopen or make patent the previously-occluded vessel.

The degree of success of this procedure depends upon the physician's ability to maintain the patency (openness) of the vessel after the angioplasty procedure. It is believed that about sixty percent of the cases reocclude in six to eight weeks, as the plaque "cold flows" back into its original position within the affected vessel. The problem at hand is to provide a means for maintaining the patency of the vessel after a "successful" angioplasty procedure.

U.S. Patent No. 4,413,989, incorporated herein by reference, discloses an angioplasty catheter assembly or catheter attachment. This patent discusses the problems associated with the angioplasty technique, yet fails to recognize and provide a solution to the reocclusion of the expanded vessel.

U.S. Patent No. 4,190,909 discloses a prosthetic graft for the repair of ducts in a living body wherein a woven tube is disclosed which may be grafted to arterial tissue and which causes ingrowth of tissue into the graft to create a permanent bond.

U.S. Patent No. 4,182,339 discloses an anastomotic device for receiving and connecting the free ends of flexible tubing to be connected. The devices shown in this and the above-mentioned patent are typical of the passive prosthetic devices which are used to connect or take the place of a blood vessel. In contrast, the instant invention is a prosthetic stent (a supporting device) which provides an active internal support for a blood vessel to contain and restrain possible "cold flow" of plaque that has been separated by an angioplasty procedure. The subject stent is a tubular body of braided filaments which provides a working component which supplements the strength of the blood vessel. In addition to support, the prosthetic stent of the instant invention provides braided filaments which are receptive to tissue ingrowth to provide a permanent bond after the stent is in place.

## SUMMARY OF THE INVENTION

The purpose of the instant invention is to provide a prosthetic stent which may be collapsed for insertion into a bodily duct such as a blood vessel via a catheter and then may be expanded to provide active internal support for the blood vessel. To accomplish this purpose, the instant invention provides a flexible, generally tubular body of braided filaments having means for biasing said tubular body toward an expanded configuration within a blood vessel. Specifically, the present invention provides a prosthetic stent comprising: a flexible, generally tubular body of braided filaments, said body having a first configuration that is radially expanded and axially shortened, and having a second configuration that is radially contracted and axially lengthened; and means for biasing said tubular body from said second configuration towards said first configuration.

One embodiment of the instant invention provides biasing of the tubular body by the braided filaments having been annealed in a radially expanded and axially shortened configuration. In another embodiment, biasing may be accomplished by the utilization of a plurality of warp filaments which are longitudinally woven into the braided filaments generally parallel to the axis of the tubular body. The warp filaments may provide a frictional interface with respect to the braided filaments to resist radial contraction and axial lengthening of the tubular body. In this embodiment, the prosthetic stent is expanded into position by inflation of the angioplasty balloon during the angioplasty procedure. In another embodiment, the warp filaments are shrinkable to axially shorten the tubular body to the radially expanded configuration. It is also within the scope of the invention to have the warp filaments elastically shrinkable or heat-recoverable.

## DESCRIPTION OF THE DRAWING

Figure 1 is a cross-sectional view of an occluded blood vessel in the body.

Figure 2 is a figure similar to Figure 1 illustrating an angioplasty technique wherein a catheter with a balloon attached is introduced into the body in the area of the occlusion.

Figure 3 illustrates the placement of the prosthetic stent of the instant invention via a catheter into an area that has been previously treated as in Figure 2 by the angioplasty procedure.

Figure 4 illustrates an alternate embodiment of the instant invention wherein the prosthetic stent further includes a plurality of warp filaments longitudinally woven into the braided-filaments shown in Figure 3 generally parallel to the tubular body of the stent. Also shown in Figure 4 is the integration of such an alternative embodiment and an angioplasty balloon as a single-use system.

Figure 5 illustrates yet another embodiment of the instant invention wherein the diameter of the prosthetic stent may vary along the length thereof.

## DESCRIPTION OF THE PREFERRED EMBODIMENTS

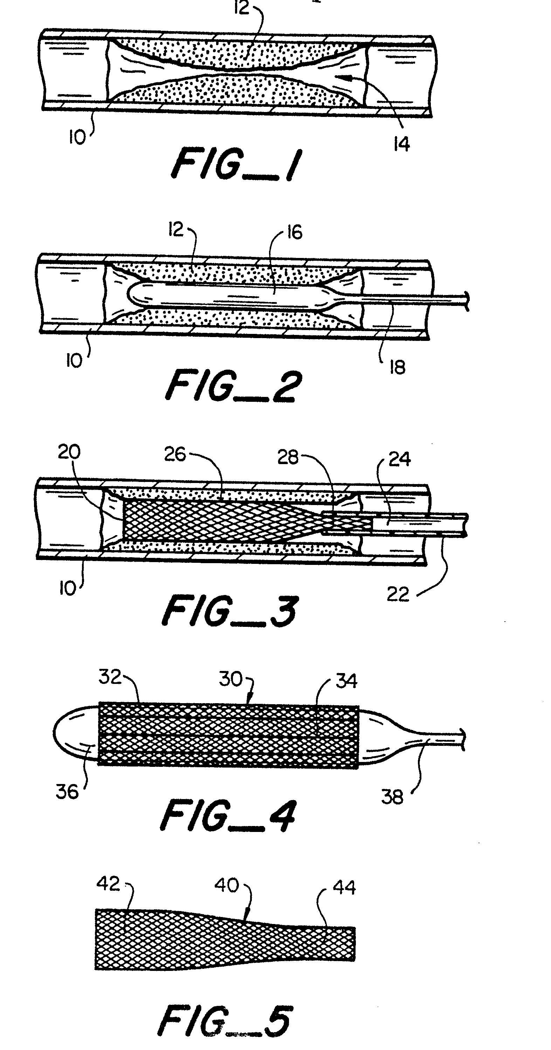

With reference to the drawing, Figure 1 illustrates a blood vessel 10 in a body and in particular in the heart. Vessel 10 is occluded (blocked) by a stenosis in the form of plaque 12 which restricts the flow of blood shown generally at 14.

Figure 2 illustrates an angioplasty procedure wherein angioplasty balloon 16 attached to catheter 18 has been inserted into the occluded area of the blood vessel 10 and the balloon 16 has been expanded to compress the plaque 12. As discussed earlier, the balloon 16 is inflated using a radioque liquid, causing the plaque 12 to separate and, after two or three cycles of inflation and deflation, the plaque is displaced sufficiently to reopen or make patent the previously-occluded blood vessel 10.

Figure 3 illustrates the prosthetic stent of the instant invention being introduced or positioned in the affected blood

0183372
MP0984-EPC

vessel 10 by a catheter 22 having a piston-like plunger 24 therein.. Prosthetic stent 20 may be collapsed for insertion into the blood vessel via the catheter 22 and then expanded to provide active internal support to the vessel 10 to prevent the compressed plaque from "cold flowing" back to its original position within the affected vessel.

What is proposed is a means of maintaining the patency of the vessel reopened using angioplasty procedure by the secondary placement via a catheter 22 of the prosthetic stent 20. A stent is defined as "...a device or mold of any suitable material used to hold a graft in place or to provide support for tubular structures that are being anastomosed." Anastomosis is "[T]o create a communication between two vessels, a passage..." These definitions were obtained from Dorland's "Illustrated Medical Dictionary," 24th Edition (1965), W. B. Saunders Co., Philadelphia, PA.

The prosthetic stent of the instant invention comprises a flexible, generally tubular body of braided filaments, the body having a first configuration that is radially expanded and axially shortened such as the portion 26 of the stent 20 shown in Figure 3, and having a second configuration that is radially contracted and axially lenthened such as the portion 28. The prosthetic stent also includes means for biasing the tubular body from the second configuration towards the first configuration. In the embodiment shown in Figure 3, this means for biasing the tubular body is integral with the braided filament construction in that the braided filaments have been annealed in the radially expanded and axially compressed first configuration to a rest geometry, as will be more fully
discussed later.  Figure 3 illustrates how the braided tubular
body may be axially lengthened and therefore radially
contracted to fit within catheter 22.  Stent 20 will then
return to the radially expanded axially compressed condition to
provide an active support for the blood vessel 10.

The prosthetic stent is lengthened and placed inside a
catheter while the stent is in its second configuration, and
positioned within the artery or vessel in the area of the original stenosis.  Once the distal end of the catheter is in
position, the prosthetic stent is pushed, via an internal
piston component 24, from the end of the catheter 22 to expand
and thus support the plaque and prevent flow that would
reocclude the vessel.

Prosthetic stent 20 of the instant invention is manufactured from virgin thermoplastic polyester fiber filaments which
are braided into a generally tubular body.  The resulting
structure resembles a "Chinese finger handcuff" device.  A
series of interwoven fibers are arranged helically and configured into a tubular shape.  Each fiber is capable of independent angular rotation which results in a contraction or
expansion of the diameter of the tube.

The fibers are oriented by heat-setting or annealing in
the expanded configuration of the tube, which results in the
tube's "rest" geometry being expanded.  The resultant tubular
product can also be compressed radially to a reduced diameter
for insertion.  The prosthetic stent, due to its helically

0183372
MP0984-EPC

oriented and radially biased filaments, possesses a remarkable "kink" resistance which makes it suitable as an internal reinforcement device for blood vessels or bodily passages and ducts to aid in maintaining patency of the vessel, passage or duct. This "kink" resistance is especially evident when the prosthetic stent is bent to a radius equal to its own diameter or less. Additionally, the construction of the prosthetic stent, in particular the helically oriented filaments, permits various diameter changes along the length of the stent with equally good "kink" resistance. This could be especially valuable in the internal reinforcement of a vessel, passage or duct whose diameter varies over relatively short lengths. Such a stent is shown in Figure 5 and will be discussed below.

In general, when a stent of the instant invention is shortened longitudinally, there is an attendant increase or expansion in the diameter as the length decreases. When released, the device returns to its normal "rest" geometry, i.e., longer length and smaller diameter. If, however, the material is held in the longitudinally compressed state at its larger diameter, supported over a mandrel, and annealed at an elevated temperature, the "rest" geometry is now the larger-diameter-shorter-length configuration, but can be compressed radially or tensioned longitudinally to the original smaller diameter.

The fibers for the instant invention are made from biocompatible materials, some examples of which are: thermoplastic polyester, polyethylene, thermoplastic soft segment polyurethane, polymethylmethacrylate, polytetrafluoroethylene, silicone polymers and elastomeric polyurethane polymers.

0183372
MP0984-EPC

The materials chosen for the prosthetic stent are polymeric substances that will not promote thrombis or blood
clotting on their surfaces, i.e. the braided filaments are non-
thrombogenic. In addition, testing has been accomplished in
the areas of cytotoxicity, direct contact and extraction hemolysis, with negative results, i.e. the braided filaments are
non-toxic and non-hemolytic. These tests indicate further the
biocompatible nature of the materials chosen.

In addition to the biocompatibility of these materials,
their geometric configuration of high open surface area allows
an interlacing of the surrounding tissue (endothelium in the
case of blood vessels) around and through prosthetic stents
made from these materials.

The ingrowth of tissue, primarily endothelium, which is
the interior lining of the blood vessel, will eventually encapsulate the prosthetic stent and the contained plaque. The
natural reconstruction of the affected area would then be
underway.

An alternate embodiment of the instant invention is the
manufacture of a prosthetic stent from bioerodable materials,
for example (but not limited to) catgut, in situations where
the necessity for permanent implantation is not indicated in
the therapeutic protocol.

Another alternate embodiment of the instant invention is
shown in Figure 4 and includes warp filaments that run longitudinally to the axis of the tubular body of the prosthetic stent

0183372

MP0984-EPC

and are interwoven with braided filaments of the stent. As can be seen clearly in Figure 4, prosthetic stent 30 comprises a generally tubular body of braided filaments 32 and longitudinally-oriented warp filaments 34 interwoven into the braided filaments 32 generally parallel to the axis of the tubular body. Warp filaments 34 frictionally interface with respect to the braided filaments to maintain the prosthetic stent in whichever configuration the stent is manipulated. Once manipulated to a radially expanded configuration, the warp filaments 34 will bias the tubular body toward this expanded configuration. In this alternate embodiment, the braided filaments need not be annealed as described earlier. The prosthetic stent may then be placed on the balloon 36 which is attached to the catheter 38, and the entire assembly is then positioned via the catheter 38 at the stenotic site. The balloon and the prosthetic stent 30 are expanded within the plaque in the one catheterization, as is shown in Figure 4. The balloon 36 may then be deflated and removed, with the prosthetic stent 30 in the expanded (second) configuration remaining, the warp filaments 34 biasing the tubular body toward the first (expanded) configuration. The expanded prosthetic stent will restrain the possible "cold flow" of the plaque.

In yet another embodiment of the instant invention, the warp filaments 34 exactly as shown in Figure 4 may be shrinkable. It is apparent from the above discussion that a shrinkable warp filament will physically attempt to move and thus bias the tubular body from the second toward the first configuration upon shrinking. It is within the scope of the

**0183372**
MP0984-EPC

invention to have this shrinking accomplished by elastic warp filaments. It is also within the scope of the invention to have yet another embodiment wherein the shrinkable warp filaments are heat-recoverable. In these embodiments the warp filaments should be secured at their ends to respective ends of the tubular body such as by heat-sealing, adhesives, etc. It is within the scope of the invention to not attach the warp filaments at their respective ends but rather to depend upon the frictional interface between the warp filaments and the braided filaments to bias and move the braided filaments. Examples of shrinkable elastic materials are synthetic latex fibers and isoprene latex fibers. Examples of shrinkable heat-recoverable materials used for making shrinkable warp filaments are: vestenamer, polyethylene oxide and polymethacrylate. These warp filaments can be heat-shrinkable at 37°C or passive to allow diametric changes and maintain these changes by external manipulation.

Figure 5 discloses a prosthetic stent 40 wherein the generally tubular body varies in diameter along the axis thereof from a large-diameter portion 42 to a smaller-diameter portion 44. This may be accomplished by known braiding techniques or by annealing a previously cylindrical stent over a non-cylindrical mandrel. Other geometries are thus within the scope of the invention.

It is therefore apparent that the invention is comprised basically of a flexible, generally tubular body of braided filaments which may take various forms described herein. However, the invention, in its broader aspects, is not limited

**0183372**
MP0984-EPC

to these specific embodiments, and departures may be made therefrom within the scope of the accompanying claims without departing from their principles and without sacrificing their chief advantages.

CLAIMS:

1.    A prosthetic stent which may be collapsed for insertion into a bodily duct and then expanded to provide active internal support to the duct, comprising:

a flexible, generally tubular body of braided filaments, said body having a first configuration that is radially expanded and axially shortened, and having a second configuration that is radially contracted and axially lengthened; and

means for biasing said tubular body from said second configuration towards said first configuration.

2.    A stent as in claim 1 wherein said means for biasing comprises braided filaments which are annealed to a rest geometry in the first configuration.

3.    A stent as in claim 1 or 2, wherein said means for biasing comprises a plurality of warp filaments longitudinally woven into the braided filaments generally parallel to the axis of said tubular body.

4.    A stent as in claim 3 wherein the warp filaments frictionally interface with respect to the braided filaments when the tubular body is in said first configuration.

5.    A stent as in claim 3 or 4, wherein the warp filaments are shrinkable.

6.    A stent as in claim 3, 4 or 5, wherein the warp filaments are shrinkable and are secured at their ends to the respective ends of the tubular body.

7. A stent as in any one of claims 3 to 6 wherein the warp filaments are elastic, and/or heat recoverable.

8. A stent as in any preceding claim, wherein said braided filaments are receptive to tissue ingrowth.

9. A stent as in any preceding claim, wherein said braided filaments are non-thrombogenic, or non-hemolytic.

10. A stent as in any preceding claim, wherein the diameter of the tubular body varies along the axial length thereof.

11. A stent as in any preceding claim wherein said braided filaments are bioerodable, and preferably are made from catgut material.

12. A stent as in any preceding claim, wherein the braided filaments are made from a thermoplastic polyester material, or from a thermoplastic soft segment polyurethane material.

13. A prosthetic stent according to any preceding chain, for use in a method of providing internal support to a bodily duct, in which method the stent is collapsed to insert it into the bodily duct and then expanded to provide active internal support for the duct.

§§§§§§§§§§§§§§§§§§§§§§§§§§§§§§§§§§§§§§§§§§§§§§§§§§§§§§§§§§§§§§§

0183372

FIG_1

FIG_2

FIG_3

FIG_4

FIG_5

| | DOCUMENTS CONSIDERED TO BE RELEVANT | | EP 85307528.1 |
|---|---|---|---|

| Category | Citation of document with indication. where appropriate, of relevant passages | Relevant to claim | CLASSIFICATION OF THE APPLICATION (Int Cl 4) |
|---|---|---|---|
| X | <u>WO - A1 - 83/03 752</u> (H.I. WALLSTEN) | 1,3,4, 7-9,12 13 | A 61 M 29/00 A 61 F 2/04 |
| A | * Totality, especially page 1; page 5, lines 11-17; page 9, lines 17-31; page 17, lines 2-11; claim 3 * | 2,5,6 | |
| | -- | | |
| X | <u>US - A - 3 868 956</u> (R.J. ALFIDI et al.) | 1,9,12 13 | |
| A | * Totality, especially fig. 14,15; column 8, lines 19-30 * | 8 | |
| | -- | | |
| A | <u>GB - A - 1 205 743</u> (NAT.RES.DEV. CORP.) | 1,13 | |
| | * Totality * | | |
| | -- | | **TECHNICAL FIELDS SEARCHED (Int. Cl.4)** |
| A | <u>US - A - 4 434 797</u> (T. SILANDER) | 1,13 | |
| | * Totality * | | A 61 B 17/00 |
| | -- | | A 61 B 19/00 |
| A | <u>US - A - 4 448 195</u> (H.H. LEVEEN) | | A 61 F 2/00 |
| | * Totality * | | A 61 M 25/00 |
| | ---- | | A 61 M 29/00 |

| The present search report has been drawn up for all claims | | |
|---|---|---|
| Place of search VIENNA | Date of completion of the search 20-02-1986 | Examiner LUDWIG |